(19) Europäisches Patentamt / European Patent Office / Office européen des brevets

(11) **EP 3 365 426 B1**

(12) **FASCICULE DE BREVET EUROPEEN**

(45) Date de publication et mention
de la délivrance du brevet:
**30.03.2022 Bulletin 2022/13**

(21) Numéro de dépôt: **16809917.4**

(22) Date de dépôt: **21.10.2016**

(51) Classification Internationale des Brevets (IPC):
*C12M 3/06* (2006.01)     *C12M 1/00* (2006.01)
*C12M 1/26* (2006.01)     *C12M 1/42* (2006.01)

(52) Classification Coopérative des Brevets (CPC):
**C12M 23/16**

(86) Numéro de dépôt international:
**PCT/FR2016/052728**

(87) Numéro de publication internationale:
**WO 2017/068296 (27.04.2017 Gazette 2017/17)**

(54) **DIMENSIONNEMENT D'UN DISPOSITIF MICROFLUIDIQUE POUR CONFINER UN ÉCHANTILLON**

DIMENSIONIERUNG EINER MIKROFLUIDISCHEN VORRICHTUNG ZUR EINGRENZUNG EINER PROBE

SIZING OF A MICROFLUIDIC DEVICE FOR CONFINING A SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.10.2015 FR 1560021**

(43) Date de publication de la demande:
**29.08.2018 Bulletin 2018/35**

(73) Titulaires:
• **CENTRE NATIONAL DE LA RECHERCHE SCIENTIFIQUE (CNRS)**
**75016 Paris (FR)**
• **Université Grenoble Alpes**
**38400 Saint-Martin-d'Hères (FR)**

(72) Inventeurs:
• **HONEGGER, Thibault**
**38000 Grenoble (FR)**
• **MAISONNEUVE, Benoît**
**07200 Fons (FR)**

(74) Mandataire: **Regimbeau**
**20, rue de Chazelles**
**75847 Paris Cedex 17 (FR)**

(56) Documents cités:
**WO-A1-00/50172     WO-A2-2007/008609**
**US-B1- 6 653 124**

• **DATABASE WPI Week 201247 Thomson Scientific, London, GB; AN 2012-H53276 XP002761483, & JP 2012 120474 A (DAINIPPON PRINTING CO LTD) 28 juin 2012 (2012-06-28)**

**Description**

**Domaine technique**

La présente invention se rapporte à un procédé de dimensionnement d'un dispositif microfluidique pour confiner un échantillon.

Dans la présente invention, deux types d'échantillons sont à envisager. Ainsi, l'échantillon à confiner peut consister :

- soit en une population de cellules (par exemple dans des neurones et des cellules eucaryotes en suspension dans un milieu de culture cellulaire, l'échantillon étant alors un échantillon biologique) ou de microparticules en suspension dans un milieu fluide porteur,
- soit un échantillon biologique consistant en au moins un explant contenu dans un milieu fluide de culture cellulaire.

Par échantillon biologique, on entend, au sens de la présente invention, un échantillon qui comporte des informations génétiques et qui est capable de se reproduire lui-même ou d'être reproduit dans un système biologique.

Une des grandes difficultés en neuro-ingénierie est le contrôle du positionnement des corps cellulaires (soma) de neurones en population (n>100 cellules/ml) en termes de concentration, de positionnement spatial et d'uniformité de positionnement de chaque soma. Cette difficulté limite sérieusement les études *in vitro* de réseaux de neurones, et plus généralement l'étude des rapports structure/fonctions du cerveau.

Il est connu de l'homme de l'art plusieurs techniques de positionnement de soma pour culture de cellules En particulier, une technique les plus répandue consiste notamment à utiliser des puces microfluidiques pour le positionnement puis la culture de neurones, mais sans contrôle précis sur le positionnement et la densité des cellules, limitant ainsi son intérêt [1]-[3].

Par ailleurs, il est aussi connu de l'homme de l'art certains types de puces microfluidiques connectant deux populations de neurones [4], [5], alors que d'autres ne connectent pas de populations de neurones entre elles, en se contentant de séparer le corps des cellules et des axones[1]-[3].

Une deuxième technique de culture de neurones utilise des puces microfluidiques mettant en œuvre des micro-piliers permettant d'isoler les neurones à une interface[6] Bien que le positionnement des micro-piliers permette le positionnement des cellules, les neurones sont positionnés individuellement, empêchant donc la culture de populations entières à moyenne ou haute densité, ce qui limite l'intérêt de cette technique.

Une troisième technique de culture de neurones utilise des billes de silicones, sur lesquelles sont déposés les neurones. L'assemblage de ces billes permet de construire des réseaux de neurones. Toutefois, avec une telle technique, les quantité densité des neurones sont limitées, l'architecture du réseau n'est pas contrôlée et les corps cellulaires ne sont pas séparés par des axories[6], [8].

Un quatrième technique consiste à réaliser la culture des neurones dans des échafaudages adaptés qui peuvent être séparés en configuration microfluidique par écoulements laminaires de ces échafaudages suivi de gélation[9]. Ces techniques sont toutefois limitées car il est peu envisageable de faire de la co-culture cellulaire à cause d'homogénéité du fluide dans la culture, limitant ainsi la taille accessible du réseau final.

Enfin, une cinquième technique consiste à réaliser la culture des neurones dans des microchambres, donnant ainsi des neurosphéroïdes, puis d'assembler ces neurosphéroïdes en un bloc de tissu pour en faire à nouveau la culture, avant de l'assembler à d'autres blocs. Cette technique présente toutefois l'inconvénient d'être longue et de nécessiter plusieurs niveaux de cultures. En outre, elle ne permet pas de contrôler la densité des neurones et des séparer les corps cellulaires des axones[10].

JP 2012 120474 divulgue aussi un dispositif microfluidique pour la culture de cellules, comportant :- une zone d'entrée (31) ;- une zone de confinement (21) correspondant à une chambre (5) disposée entre le premier canal (2) et le second canal (3) où au moins une partie de l'échantillon est confiné et une zone de sortie (33) adaptée pour évacuer le liquide avec un premier canal reliant la zone d'entrée et la zone de confinement.

L'objectif de la présente invention est de pallier les inconvénients précités en permettant le positionnement contrôlé de corps cellulaires de neurones, primaires ou de lignée, à l'échelle de populations entières, dans des chambres microfluidiques, à des densités surfaciques ou volumiques contrôlées.

A cette fin, le demandeur a mis au point un procédé de dimensionnement d'un dispositif microfluidique (ou puce microfluidique) permettant d'optimiser l'écoulement de cellules telles que des neurones en suspension dans un milieu fluide porteur.

Dans le cas où l'échantillon à confiner consiste en une population de cellules ou de microparticules en suspension dans un milieu fluide porteur, on utilisera un dispositif microfluidique (ou puce microfluidique) comportant :

- une zone d'entrée adaptée pour recevoir le milieu fluide porteur contenant l'échantillon, ladite zone d'entrée correspondant à un réservoir cylindrique d'entrée de diamètre $D_{in}$,

- une zone de confinement (ou chambre de déposition) dans laquelle au moins une partie de l'échantillon est confinée comprenant un fond de surface $S_{ch}$ et de longueur $L_{ch}$ et une paroi latérale de hauteur $H_{ch}$, ladite zone de confinement communiquant avec ladite la zone d'entrée par un premier canal de longueur $L_{in}$, de hauteur $H_{in}$ et de largeur $W_{in}$, et
- une zone de sortie adaptée pour évacuer ledit liquide incluant l'échantillon, ladite zone de sortie correspondant à un réservoir cylindrique de sortie de diamètre $D_{out}$, ladite zone de sortie communiquant avec ladite zone de confinement par un second canal de longueur $L_{out}$, de hauteur $H_{out}$ et de largeur $W_{out}$.

De manière avantageuse, la zone de confinement peut présenter une géométrie cylindrique avec un fond circulaire de diamètre $D_{ch}$, de sorte que $L_{ch} = D_{ch}$. Mais, d'autres géométries sont possibles.

Dans ce cas, la présente invention a donc pour objet un procédé pour dimensionner un tel dispositif microfluidique comportant les étapes suivantes :

A. dimensionnement de ladite zone de confinement en fonction de la quantité souhaitée de cellules ou de microparticules à confiner et du taux de recouvrement $\phi$ souhaité du fond de ladite zone de confinement par lesdites cellules ou microparticules, de manière à définir la surface $D_{ch}$ et la hauteur $H_{ch}$ caractérisant la zone de confinement;
B. dimensionnement du premier canal et du second canal comprenant :

- b1) le calcul de la vitesse de sédimentation $v_{sedi}$ d'une particule ou d'une cellule,
- b2) détermination de la vitesse $v_{ch}$ du milieu fluide porteur dans ladite zone de confinement en fonction de la vitesse de sédimentation $V_{sedi}$ d'une particule ou d'une cellule conformément à l'équation (1) :

$$V_{ch} \leq V_{sedi} \frac{H_{ch}}{D_{ch}} \qquad (1)$$

- b3) détermination de la perte de charge dans ledit dispositif en fonction du volume de milieu fluide injecté $\Delta Z$ entre les zones d'entrée et de sortie qui est nécessaire à l'établissement d'un écoulement adéquat dans la zone de confinement;
- b4) détermination des paramètres géométriques dudit dispositif microfluidique à partir de $\Delta Z$ et de la vitesse $v_{ch}$ du milieu fluide porteur.

Par paramètres géométriques du dispositif microfluidique, on entend, au sens de la présente invention, les paramètres $D_{in}$, $H_{in}$, $W_{in}$, $L_{in}$, $D_{out}$, $H_{out}$, $L_{out}$ et $W_{out}$ mentionnés précédemment et qui caractérisent la forme et les dimensions du dispositif microfluidique.

Par écoulement adéquat, on entend, au sens de la présente invention, un écoulement permettant la déposition des cellules ou des particules dans la zone de confinement, sans les entrainer vers ladite zone de sortie ou en minimisant la perte de cellules écoulées dans la zone de sortie. Un écoulement adéquat est ici imposé par une différence de volume entre le réservoir d'entrée et le réservoir de sortie. Pour permettre l'écoulement, il est également possible d'utiliser des systèmes de pompage extérieurs.

La première étape du procédé selon l'invention est l'étape de dimensionnement de la zone de confinement (ou chambre de déposition) en fonction de la quantité souhaitée de cellules ou de microparticules à confiner et du taux de recouvrement $\phi$ souhaité du fond de la zone de confinement par les cellules ou microparticules, de manière à définir la surface $D_{ch}$ et la hauteur $H_{ch}$ caractérisant la zone de confinement.

Par taux de recouvrement $\phi$ du fond de la zone de confinement, on entend au sens de la présente invention le rapport $\phi$ entre la surface couverte par les particules (une fois déposées au fond) sur la surface totale du fond de la zone de confinement.

En d'autres termes, la taille de la zone de confinement est décidée en fonction de l'application choisie. Celle-ci peut varier de quelques dizaines de micromètres à quelques millimètres, en rayon et en hauteur.

De manière avantageuse, cette étape A de dimensionnement peut comprendre les sous-étapes suivantes :

A1) la détermination de la surface $S_{ch}$ du fond de la zone de confinement conformément à la formule de Stokes (5):

$$\phi = \frac{N \times \pi r^2}{Sch} \qquad (5)$$

avec

- $\phi$ étant le taux de recouvrement $\phi$ du fond,
- r étant le rayon d'une particule ou d'une cellule
- N étant le nombre de neurones ou de cellules à confiner, défini par l'équation:

$$N = \varphi \times V \qquad (6)$$

avec

- $\varphi$ étant la concentration en cellules ou neurones dans ledit échantillon (mesuré en début d'expérience),
- V étant le volume d'échantillon entré dans ledit dispositif microfluidique à un instant t, défini conformément à l'équation :

$$V = Q \times t \qquad (7)$$

avec
- Q étant le débit de l'échantillon dans ledit dispositif microfluidique.

A2) la fixation par l'utilisateur dudit dispositif microfluidique de la hauteur $H_{ch}$ de la paroi de hauteur $H_{ch}$, en fonction de la quantité de volume désiré dans la zone de confinement et des contraintes de fabrication associées.

La deuxième étape b2) du procédé selon l'invention est l'étape de dimensionnement des premier et second canaux. Cette étape B comprend plusieurs sous-étapes.

La première sous-étape est l'étape b1) de calcul de la vitesse de sédimentation $V_{sedi}$ d'une particule ou d'une cellule.

De manière avantageuse, la vitesse de sédimentation $V_{sedi}$ d'une particule ou d'une cellule peut être calculée conformément à l'équation de Stokes (8):

$$vsedi = \frac{2\,r^2 g \Delta\rho}{9\eta} \qquad (8)$$

avec :

- r étant le rayon d'une particule ou d'une cellule,
- $\eta$ étant la viscosité dynamique dudit milieu fluide porteur, et
- $\Delta\rho$ étant la différence de masse volumique entre celle des particules ou cellules à confiner et le milieu fluide porteur.

La deuxième sous étape est l'étape b2) de calcul de la vitesse $v_{ch}$ du milieu fluide porteur dans ladite zone de confinement. Cette détermination est réalisée, à partir de de la vitesse de sédimentation $V_{sedi}$ calculée à la sous-étape b1), selon l'équation (1) :

$$V_{ch} \leq V_{sedi} \frac{H_{ch}}{D_{ch}} \qquad (1)$$

En effet, il a été constaté qu'afin de confiner tout l'échantillon dans la zone de confinement, la vitesse $v_{ch}$ d'écoulement du milieu fluide porteur dans zone de confinement doit être égale ou inférieure à la vitesse de sédimentation $V_{sedi}$ multipliée par le quotient de la hauteur $H_{ch}$ et de la longueur $D_{ch}$ de la zone de confinement (ou chambre), conformément à l'équation 1.

L'équation (1) permet de déterminer trois régimes de fonctionnement du dispositif microfluidique selon l'invention. En prenant en compte les propriétés géométriques du dispositif microfluidique selon l'invention pour déterminer les pertes de charge (comme décrit ci-après), ainsi que les conditions d'opération (volume d'entrée de l'échantillon et concentration en cellules ou en microparticules), il est possible de concevoir le dispositif de manière qu' une partie seulement de l'échantillon biologique soit confinée dans la chambre 5 ( $V_{ch} > V_{sedi} \frac{H_{ch}}{D_{ch}}$ , ou que tout l'échantillon biologique soit confiné dans la zone de confinement comme cela est recherché dans la présente invention, de manière uniforme $(V_{ch} = V_{sedi}\frac{H_{ch}}{D_{ch}})$ ou de manière non-uniforme $(V_{ch} < V_{sedi}\frac{H_{ch}}{D_{ch}})$.

La troisième sous-étape est l'étape b3) de détermination de la perte de charge dans le dispositif microfluidique selon l'invention, en fonction du volume de milieu fluide injecté ΔZ entre les zones d'entrée et de sortie qui est nécessaire à l'établissement d'un écoulement adéquat dans la zone de confinement. Cette perte de charge est établie par la quantité de milieu fluide porteur injectée lors de l'utilisation du dispositif microfluidique selon l'invention.

La quatrième sous-étape est l'étape b4) de détermination des paramètres géométriques $D_{in}$, $H_{in}$, $W_{in}$, $L_{in}$, $D_{out}$, $H_{out}$, $L_{out}$ et $W_{out}$ dudit dispositif microfluidique à partir de ΔZ et de la vitesse $v_{ch}$ du milieu fluide porteur.

Les pertes de charges qui sont considérées pour cette détermination sont des pertes de charges régulières. On néglige, dans le cadre de la présente invention les pertes de charges singulières.

Les pertes de charge régulières sont étroitement liées aux frottements du liquide sur les parois des premier et second canaux. Pour des régimes à faible nombre de Reynolds (Re<<1) pour lesquels l'écoulement du liquide est laminaire dans tout le dispositif microfluidique, Les pertes de charges régulières pour ces régimes sont estimées, de manière générale, par l'équation générale (2) :

$$\Delta z = \frac{\lambda \eta Q L}{W H^3 \rho g} \qquad (2)$$

avec *W, H* et *L* représentant la largeur, la hauteur et la longueur des canaux, Q désignant le débit volumique du milieu fluide porteur, *g* désignant l'accélération gravitationnelle, $\eta$ et $\rho$ désignant respectivement la viscosité dynamique et la masse volumique du liquide respectivement et enfin $\lambda$ représentant un coefficient de frottement, approximé à faible nombre de Reynolds par l'équation suivante :

$$\lambda = 12(1 - 6(\frac{2}{\pi})^5(\frac{H}{W})) \qquad (3)$$

ce coefficient de frottement $\lambda$ étant calculé pour chacune des canaux du dispositif microfluidique selon l'invention.

Par ailleurs, les pertes de charges singulières sont essentiellement dues aux accidents de canalisation et ils sont liés aux changements de géométrie entre les différents éléments d'un dispositif microfluidique. Ces pertes de charges sont typiquement estimées par l'équation suivante :

$$\Delta z = \frac{K v^2}{2g} \qquad \text{(2bis)}$$

Dans cette équation, v représente la vitesse du liquide dans la section considérée, g désigne l'accélération gravitationnelle et K est un paramètre dépendant du type de perte de charge singulière. Il est à noter que le paramètre K est estimé en fonction du type d'accident de canalisation (rétrécissement brusque, élargissement brusque, coude brusque, coude arrondi etc.) et l'estimation de ce paramètre K est effectuée par des formules bien connues par l'homme du métier, qui sont différentes en fonction du type d'accident de canalisation.

Comme on peut le comprendre par les équations (2) et (2bis) mentionnées ci-dessus, les pertes de charges ne dépendent pas seulement des dimensions des canaux du dispositif microfluidique, mais aussi du débit volumique, de la viscosité dynamique et de la masse volumique du liquide pour les pertes de charges régulières, ainsi que de la vitesse du liquide dans la section considérée pour les pertes de charges singulières et du type de ces pertes de charges singulières.

La détermination des paramètres géométriques du dispositif microfluidique selon l'invention de la sous-étape b4) peut avantageusement être réalisée comme suit :

• B41) choix de sept paramètres géométriques dudit dispositif microfluidique parmi les huit paramètres géométriques $D_{in}$, $H_{in}$, $W_{in}$, $L_{in}$, $D_{out}$, $H_{out}$, $L_{out}$ et $W_{out}$ (en fonction de la géométrie de la zone de confinement) ; et

• B42) calcul du paramètre géométrique inconnu restant en fonction de ΔZ et de la vitesse $v_{ch}$ du milieu fluide porteur.

Dans le cadre de la présente invention, on négligera les pertes de charges singulières, et on part de l'hypothèse que l'écoulement du milieu fluide porteur dans le dispositif microfluidique est laminaire.

Ainsi, dans ce cadre-là (perte de charge ΔZ régulière et écoulement laminaire du milieu fluide porteur), le paramètre inconnu pourra être calculé dans la sous-étape b42) à partir de l'équation (2') dérivée de l'équation générale (2) :

$$\Delta z = \frac{\eta Q}{\rho g}\left(\frac{\lambda_{in}L_{in}}{W_{in}H_{in}^3} + \frac{\lambda_{ch}L_{ch}}{W_{ch}H_{ch}^3} + \frac{\lambda_{out}L_{out}}{W_{out}H_{out}^3}\right) \qquad (2')$$

avec :

- Q étant le débit de fluide constant dans ledit dispositif défini par l'équation (9) :

$$Q = v_{ch} \times H_{ch} \times W_{ch} \qquad (9)$$

- g désigne l'accélération gravitationnelle,
- $\eta$ désigne la viscosité dynamique du milieu fluide porteur,
- $\rho$ désigne la masse volumique du milieu fluide porteur,
- $L_{ch}$ désigne la largeur de la zone de confinement (si celle-ci présente une géométrie circulaire, alors $L_{ch} = w_{ch} = D_{ch}$)
- $\lambda$ représente un coefficient de frottement, calculé pour un faible nombre de Reynolds conformément à l'équation (3) :

$$\lambda = 12\left(1 - 6\left(\frac{2}{\pi}\right)^5\left(\frac{H}{W}\right)\right) \qquad (3)$$

Si l'on choisit que le paramètre inconnu est $L_{out}$ (les autres paramètres $D_{in}$, $H_{in}$, $W_{in}$, $L_{in}$, $D_{out}$, $H_{out}$ et $W_{out}$ étant alors fixés), celui-ci peut alors être calculé selon l'équation (4) :

$$L_{out} = \frac{W_{out}H_{out}^3}{\lambda_{out}}\left(\Delta z \frac{\rho g}{\eta Q} - \frac{\lambda_{in}L_{in}}{W_{in}H_{in}^3} - \frac{\lambda_{ch}L_{ch}}{W_{ch}H_{ch}^3}\right) \qquad (4)$$

Comme indiqué précédemment, l'échantillon à confiner consiste en une population de cellules ou de microparticules en suspension dans un milieu fluide porteur. Cet échantillon peut être soit un échantillon non biologique, soit un échantillon biologique.

Selon une première variante de ce mode de réalisation du procédé selon l'invention, l'échantillon peut être un échantillon biologique consistant en une population de cellules choisies parmi les neurones et les cellules eucaryotes en suspension dans un milieu de culture cellulaire, ou en suspension dans de l'eau, salée ou non, un solvant, un hydrogel ou un échafaudage organique ou un polymère. Avantageusement, l'échantillon biologique pourra comprendre une population de cellules allant de 100 cellules par millilitre à $10^{10}$ cellules par millilitre. Quelle que soit le type de cellules dans l'échantillon biologique, la vitesse de sédimentation des cellules doit être supérieure à la vitesse induite par ses mouvements browniens.

Selon une deuxième variante de ce mode de réalisation du procédé selon l'invention, l'échantillon peut être un échantillon non-biologique consistant en une population de microparticules en suspension dans de l'eau, salée ou non, ou dans un solvant, un hydrogel ou un échafaudage organique (par exemple de type collagène) ou un polymère, lesdites microparticules étant choisies parmi les microparticules métalliques, ou en matériau semi-conducteur, ou en polyéthylène-glycol (PEG).

De manière avantageuse, on peut relier la zone de confinement à au moins une chambre d'isolation, par au moins un canal additionnel ayant une résistance hydraulique permettant le passage, sans retour, de l'échantillon dans ladite chambre d'isolation.

De manière avantageuse, cette chambre d'isolation peut être une zone de confinement d'un dispositif microfluidique additionnel.

De manière avantageuse, cette chambre d'isolation peut comporter un échantillon additionnel. Dans ce cas, on pourra utiliser un dispositif microfluidique ainsi dimensionné pour étudier l'interaction entre l'échantillon initial placé dans la zone de confinement et l'échantillon additionnel dans la chambre d'isolation.

Le procédé de dimensionnement selon l'invention peut être utilisé dans des domaines différents. Par exemple, dans le cas où l'échantillon est un échantillon biologique, le procédé selon l'invention pourra être utilisé dans le domaine de neuroscience si l'échantillon biologique comprend une population de neurones, ou dans le domaine de cancérologie si l'échantillon biologique comprend une population de cellules cancéreuses. Dans le cas où l'échantillon est un échantillon non-biologique, le procédé selon l'invention pourra être utilisé dans le domaine de photonique si par exemple l'échantillon non-biologique comprend une population de particules métalliques.

Dans le cas où l'un échantillon est un échantillon biologique consistant en au moins un explant contenu dans un milieu fluide de culture cellulaire, on utilisera un dispositif microfluidique (ou puce microfluidique) différent de celui utilisé dans le premier mode de réalisation, spécifiquement adapté à la nature de l'échantillon. Un tel dispositif comporte :

- une zone d'entrée adaptée pour recevoir le milieu fluide de culture cellulaire incluant l'échantillon, ladite zone d'entrée correspondant à un réservoir cylindrique d'entrée de diamètre $D_{in}$,

- un premier canal de longueur $L_{in}$, de hauteur $H_{in}$ et de largeur $W_{in}$, dans lequel est aménagée une zone de confinement, ledit premier canal communiquant d'une part avec ladite zone d'entrée et d'autre part avec

- un second canal de longueur $L_{out}$, de hauteur $H_{out}$ et de largeur $W_{out}$, ledit second canal communiquant d'une part avec ledit premier canal et d'autre part avec une zone de sortie adaptée pour évacuer ledit liquide incluant l'échantillon, ladite zone de sortie correspondant à un réservoir cylindrique de sortie de diamètre $D_{out}$,

Dans ce cas, la présente invention a alors pour objet un procédé pour dimensionner un tel dispositif microfluidique comportant les étapes suivantes :

A. choix de l'explant à confiner dans ledit dispositif microfluidique,
B. dimensionnement ladite zone d'entrée de manière à pouvoir y introduire ledit explant avec la quantité de milieu fluide porteur nécessaire à sa survie,
C. dimensionnement desdits premier et deuxième canaux de manière que la largeur $W_{out}$ et/ou la hauteur $H_{out}$ du second canal soient respectivement inférieures à la largeur $W_{in}$ et/ou la hauteur $H_{in}$ du premier canal, pour qu'au moins une partie de l'échantillon soit confiné dans ladite zone de confinement du premier canal et que le milieu fluide de culture cellulaire introduit dans la zone d'entrée puisse s'écouler via le premier canal, la zone de confinement puis le second canal vers la zone de sortie.

De manière avantageuse, la largeur $W_{out}$ et/ou la hauteur $H_{out}$ du second canal peuvent être inférieures aux dimensions de l'explant.

De manière avantageuse, la largeur $w_{in}$ et/ou la hauteur $H_{in}$ du premier canal peuvent être supérieures d'au moins 1% aux dimensions de l'explant.

De manière avantageuse, on peut adapter la zone de sortie du dispositif microfluidique pour permettre une aspiration dudit milieu fluide de culture cellulaire, de sorte que l'écoulement du milieu fluide sera adéquat pour l'application envisagée.

Par écoulement adéquat, on entend, au sens de la présente invention pour ce mode de réalisation, un écoulement par aspiration en sortie. Pour faciliter l'écoulement, il est possible d'utiliser un système de pompage extérieur.

A titre d'explants utilisables comme échantillons biologique dans ce deuxième procédé de dimensionnement selon l'invention, on peut notamment citer un tissu, une rétine, un ganglion ou un hippocampe.

D'autres avantages et particularités de la présente invention résulteront de la description qui va suivre, donnée à titre d'exemple non limitatif et faite en référence aux figures annexées et aux exemples correspondants :

- La figure 1 illustre une vue schématique en coupe d'un exemple de dispositif microfluidique dimensionné selon le premier mode de réalisation du procédé de l'invention, ainsi qu'une vue de dessus de ce même dispositif ;
- La figure 2 illustre une photographie de la zone de confinement (ou chambre de déposition) du dispositif microfluidique illustré sur la figure 1 ;
- La figure 3 illustre une vue schématique en coupe et une vue schématique de dessus d'un exemple de dispositif microfluidique dimensionné selon le deuxième mode de réalisation du procédé de l'invention.
- Les figures 4 et 5 illustrent chacune une photographie de la zone de confinement du dispositif microfluidique de la figure 3, respectivement de cas où l'explant est un ganglion (figue 4) et un hippocampe (figure 5).

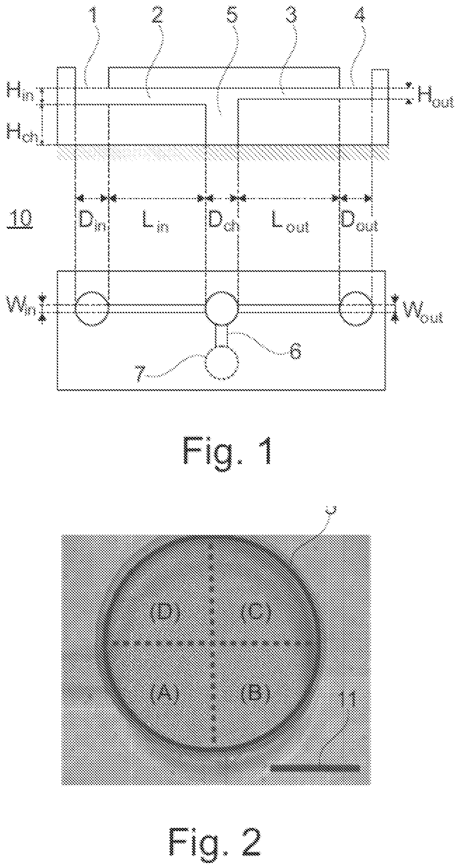

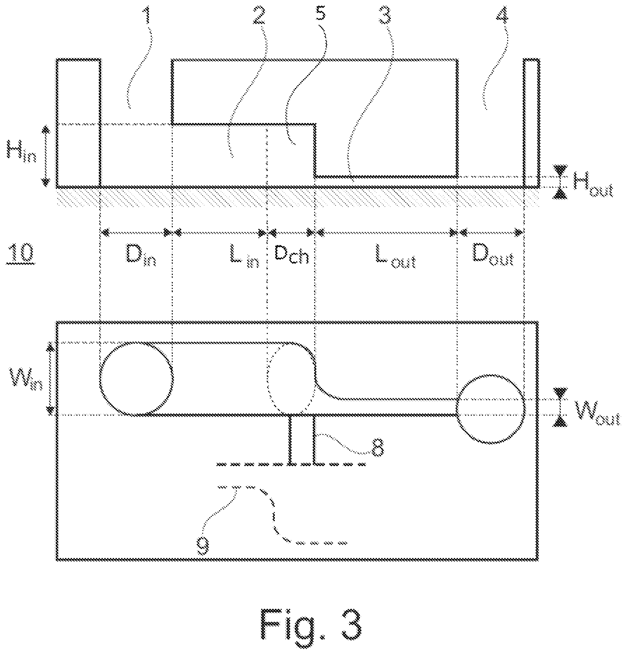

Les éléments identiques représentés sur les figures 1 à 8 sont identifiés par des références numériques identiques. Les figures 1 et 2 sont commentées de manière plus détaillée dans l'exemple 1, tandis que les figures 3 à 5 sont commentées de manière plus détaillée dans l'exemple 2.

**EXEMPLE 1** :

**Premier mode de réalisation** : dimensionnement d'une puce microfluidique (illustrée sur les figures 1 et 2) en vue de **son utilisation pour la déposition de neurones.**

Dispositif

On utilise pour la déposition de neurones un dispositif microfluidique 10, dimensionné conformément au procédé selon l'invention (premier mode de réalisation).

La figure 1 montre en particulier (en vue de profil et vue de dessus) un exemple de dispositif microfluidique 10 dimensionné selon le premier mode de réalisation du procédé de l'invention. La figure 2 montre une photographie de la chambre de déposition 5 utilisée dans le cas d'une déposition de neurones.

Ce dispositif microfluidique 10 comporte une zone d'entrée 1 adaptée pour recevoir un liquide incluant l'échantillon biologique et une zone de sortie 4 adaptée pour évacuer ce liquide. La zone d'entrée 1 et la zone de sortie 4 correspondent aux réservoirs cylindriques qui ont les mêmes diamètres et des hauteurs différentes (la hauteur de la zone d'entrée 1 est inférieure à la hauteur de la zone de sortie 4). En particulier, le diamètre $D_{in}$ du réservoir, qui correspond à la zone d'entrée 1, est le même que le diamètre $D_{out}$ du réservoir qui correspond à la zone de sortie 4. Cependant, la zone d'entrée 1 et la zone de sortie 4 peuvent avoir des diamètres différents. En outre, la zone d'entrée 1 et/ou la zone de sortie 4 peuvent également présenter d'autres formes que la forme cylindrique (par exemple des formes carrées).

Il est à noter que les dimensions (hauteur et section) de la zone d'entrée 1 sont choisies par rapport à l'échantillon biologique introduit dans la zone d'entrée 1 : il est souhaitable que les dimensions de la zone d'entrée 1 soient telles qu'elle puisse stocker la totalité des nutriments inclus dans le liquide de culture cellulaire et qui sont nécessaires à la survie des neurones dans la zone de confinement (ou chambre de déposition) pour une durée allant de 12 heures à 48 heures (mais non limité à ces durées), afin d'effectuer des études *in vitro* sur ces neurones.

Le dispositif microfluidique 10 de la figure 1 comporte en outre une zone de confinement 5 (ou chambre de déposition) où l'échantillon biologique est confiné. Plus particulièrement, dans l'exemple de la figure 1, la chambre 5 a une forme cylindrique ayant une longueur $D_{ch}$ et une hauteur $H_{ch}$. Il est à noter que dans l'exemple de la figure 1, la longueur $D_{ch}$ (qui correspond au diamètre de la chambre cylindrique) est inférieure au diamètre $D_{in}$ de la zone d'entrée 1 et au diamètre $D_{out}$ de la zone de sortie 4. Cependant, la longueur $D_{ch}$ peut être égale ou supérieure au diamètre $D_{in}$ de la zone d'entrée 1 et au diamètre $D_{out}$ de la zone de sortie 4. En outre, il est à noter que la chambre 5 peut également présenter d'autres formes, par exemple une forme allongée, triangulaire, carrée ou pentagonale.

En outre, il est à noter que les dimensions (hauteur et longueur) de la chambre 5 sont déterminées en fonction du volume de liquide reçu par la zone d'entrée 1 à confiner dans cette chambre 5, et elles peuvent varier de quelques micromètres à quelques centimètres.

Par ailleurs, la figure 1 montre que la chambre 5 est disposée entre un premier canal 2 et un second canal 3, le premier canal 2 reliant la zone d'entrée 1 et la chambre 5 et le second canal 3 reliant la chambre 5 et la zone de sortie 4. Le premier canal 2 a une hauteur $H_{in}$, une longueur $L_{in}$ et une largeur $W_{in}$ et le second canal 3 a une hauteur $H_{out}$, une longueur $L_{out}$ et une largeur $W_{out}$. Le premier et le second canal ont des hauteurs inférieures à celles de de la zone d'entrée 1 et de la zone de sortie 4, et ils sont disposés l'un par rapport à l'autre de telle sorte que le liquide reçu par la zone d'entrée 1 s'écoule via le premier canal 2, la chambre 5 puis le second canal 3 vers la zone de sortie 4.

Il est à noter que l'écoulement du liquide dans le dispositif microfluidique de la figure 1 peut être stoppé à tout moment soit en retirant le volume de liquide restant de la zone d'entrée 1, soit en rajoutant un volume de liquide équivalent dans la zone de sortie 4.

En outre, dans l'exemple de la figure 1, la hauteur $H_{in}$ et la largeur $W_{in}$ du premier canal 2 sont supérieures à la hauteur $H_{out}$ et la largeur $W_{out}$ respectives du second canal 3 et la longueur $L_{in}$ du premier canal 2 est le même que la longueur $L_{out}$ du second canal 3. Il est à noter que dans un autre exemple, la hauteur $H_{in}$ et la largeur $W_{in}$ du premier canal 2 peuvent être égales ou inférieures à la hauteur $H_{out}$ et la largeur $W_{out}$ respectives du second canal 3, et la longueur $L_{in}$ du premier canal 2 peut être supérieure ou inférieure à la longueur $L_{out}$ du second canal 3.

Il a été constaté que la présence du premier canal 2 en amont et du second canal 3 en aval de la zone de confinement (chambre 5) dans le dispositif microfluidique 10 de la figure 1 (les termes « en amont » et « en aval » sont déterminés par rapport au sens de l'écoulement mentionné ci-dessus du liquide qui est reçu par la zone d'entrée 1), et plus particulièrement l'adaptation adéquate des dimensions (i.e. la hauteur, la longueur et la largeur) de ces deux canaux, permet de confiner au moins une partie de l'échantillon biologique dans la zone de confinement en contrôlant la distribution spatiale de l'échantillon biologique confiné dans cette zone de confinement. En particulier, il a été constaté qu'une adaptation des dimensions respectives du premier canal 2 et du second canal 3 en fonction de la perte de charge dans le dispositif microfluidique 10 permet d'obtenir un dispositif microfluidique 10 dans lequel on peut contrôler la vitesse de l'écoulement du liquide dans la chambre 5, et le contrôle de cette vitesse permet le contrôle de la distribution spatiale de l'échantillon biologique dans cette chambre 5 lors de l'écoulement du liquide. Il est à noter que la vitesse de l'écoulement du liquide dans la chambre 5 dépend de la différence entre le volume de liquide dans la zone d'entrée 1 et le volume de liquide dans la zone de sortie 4 du dispositif microfluidique 10. Ainsi, le contrôle de la vitesse de l'écoulement du liquide dans la chambre 5 peut être effectué en contrôlant cette différence des volumes du liquide entre la zone d'entrée 1 et la zone de sortie 4. Le contrôle de cette différence des volumes du liquide peut être effectué en rajoutant ou en retirant un volume de liquide soit dans la zone d'entrée 1 soit dans la zone de sortie 4.

Echantillon

L'échantillon mis en œuvre dans cet exemple est un milieu de culture de neurones, qui comprend :

- $5.10^7$ neurones/mL, en suspension dans
- un milieu liquide de culture cellulaire, contenant du Neurobasal, du b27, de la L-cystéine et du Pen/Strep.

Dimensionnement de la puce microfluidique des figures 1 et 2 en fonction de l'échantillon

Un exemple de confinement d'une population de neurones dans la chambre de déposition 5 du dispositif microfluidique 10 de la figure 1 est illustré par la photographie de la figure 2 : dans cette puce 10, les diamètres de la zone d'entrée 1 et de la zone de sortie 4 ne sont pas les mêmes.

En particulier, la photographie de la figure 2 illustre une barre d'échelle 11 d'une longueur de 200 pm et des cellules qui sont confinées dans la chambre 5 (zone de confinement). Ces cellules sont des neurones de dimensions d'environ $10\mu m$ et d'une densité surfacique de 95%.

Comme le montre la figure 2, la zone de confinement est séparée entre quatre zones (voir les zones (A), (B), (C), (D) dans la figure 2).

Comme illustré sur la figure 2, les neurones recouvrent uniformément la surface de la zone de confinement. Il est à noter qu'afin d'obtenir ce confinement uniforme dans la zone de confinement, les dimensions du premier canal et du second canal ont été adaptées afin de fournir un dispositif microfluidique 10 dans lequel la vitesse $V_{ch}$ d'écoulement du liquide dans la chambre 5 peut être contrôlée. En particulier, dans le cas du confinement uniforme de l'exemple de la figure 2, la vitesse $V_{ch}$ d'écoulement du liquide dans la chambre 5 est contrôlée afin d'être égale ou inférieure à la vitesse de sédimentation $V_{sedi}$ de l'échantillon biologique multipliée par le quotient de la hauteur $H_{ch}$ et de la longueur $D_{ch}$ de la chambre 5 (conformément à l'équation 1).

Il est à noter que cette adaptation des dimensions du premier canal et du second canal a été fait en prenant en compte les pertes de charge des équations (2') et (4) mentionnés ci-dessus, la concentration et les dimensions des neurones ainsi que les dimensions de la zone d'entrée 1, de la zone de sortie 4 et de la chambre 5.

En particulier, la puce microfluidique 10 illustrée sur la figure 1 présente les caractéristiques suivantes :

- le canal d'entrée 2 a une hauteur $H_{in}$ de 100 $\mu m$, une largeur $W_{in}$ de 120 pm et une longueur $L_{in}$ de 1500 pm ;
- le canal de sortie 3 présente une hauteur $H_{out}$ de 20 $\mu m$, une largeur $W_{out}$ de $120\mu m$;
- en outre, la chambre de déposition 5 est également cylindrique et a une hauteur de 100 pm et un diamètre de 500 pm ;
- par ailleurs, la zone d'entrée 1 est cylindrique ; elle présente une hauteur $D_{in}$ de 5 mm et un diamètre de 4 mm, et la zone de sortie 4 est aussi cylindrique et présente une hauteur de 4 mm et un diamètre $D_{out}$ de 4,5 mm.

On calcule $L_{out}$ conformément au procédé de dimensionnement selon l'invention, en partant de l'équation (2').

Dans le présent exemple, les paramètres fixés au départ étaient les paramètres $D_{in}$, $H_{in}$, $W_{in}$, $L_{in}$, $D_{out}$, $H_{out}$, et $W_{out}$.

Mais, il est également possible de calculer un autre paramètre inconnu, différent de $L_{out}$, et de fixer les sept autres paramètres restants.

La vitesse de l'écoulement du liquide de culture cellulaire dans la chambre de déposition 5 est de $41 \cdot 10^{-5}$ m/s et cette vitesse a été obtenue par l'introduction d'un volume de liquide de 20 pL dans la zone d'entrée 1.

Il est à noter que dans le dispositif microfluidique de la publication de Taylor et *al.* [1], il n'existe pas un premier canal et un second canal reliés avec la zone de confinement de Taylor (chambre de culture), comme c'est le cas dans le dispositif microfluidique 10 dimensionné conformément à la présente invention, et ainsi. Dans le dispositif de Taylor, il n'est pas possible de confiner l'échantillon dans la zone de confinement en contrôlant la distribution spatiale de l'échantillon dans cette zone de confinement.

Le contrôle de la vitesse de l'écoulement du liquide dans la chambre 5 permet le contrôle de la distribution spatiale de l'échantillon biologique dans cette chambre 5 lors de l'écoulement du liquide.

Protocole expérimental

La culture de cellules exige un milieu stérile, pour éviter toute forme de contamination. Les puces microfluidiques une fois assemblées n'étant pas stériles, il convient donc de les stériliser avant utilisation.

A cette fin, la puce microfluidique 10 utilisée dans cet exemple pour la déposition de neurones (et dont la chambre de déposition 5 est illustrée par la figure 2), est introduite dans un environnement stérile (telle qu'une hotte à flux laminaire stérile), et de l'éthanol est introduit dans la zone de chargement pour remplacer l'eau dans les canaux 2, 3 et dans la chambre de déposition 5. L'éthanol est ensuite rincé avec de l'eau stérile trois fois, avant d'exposer la puce 10 aux UV pendant 30 minutes.

La surface de la chambre de déposition peut ensuite être fonctionnalisée pour promouvoir la culture des cellules.

Une fois la puce 10 prête, un volume prédéfini de l'échantillon (de 0,5 à 10 mL) est déposé dans la zone de chargement (Zone d'entrée 1) pour générer l'écoulement et commencer la déposition de cellules.

L'écoulement peut être stoppé à tout moment, soit en retirant le volume restant dans la zone de chargement, soit en rajoutant un volume équivalent dans la zone de sortie (égalisant ainsi la pression hydrostatique entre l'entrée et la sortie).

## EXEMPLE 2 :

**Deuxième mode de réalisation : dimensionnement d'une puce microfluidique (illustrée sur les figures 3 à 5 2) en vue de son utilisation pour la déposition d'explants.**

Dispositif

On utilise pour la déposition d'explants un dispositif microfluidique 10, dimensionné conformément au procédé selon l'invention (deuxième mode de réalisation).

La figure 3 montre en particulier (en vue de profil et vue de dessus) un exemple de dispositif microfluidique 10 dimensionné selon le deuxième mode de réalisation du procédé de l'invention.

Comme illustré sur la figure 3, le dispositif microfluidique 10 comporte une zone d'entrée 1 adaptée pour recevoir un liquide incluant l'échantillon biologique et une zone de sortie 4 adaptée pour évacuer ce liquide. La zone d'entrée 1 et la zone de sortie 4 correspondent aux réservoirs cylindriques qui présentent les mêmes diamètres et les mêmes hauteurs. En particulier, le diamètre $D_{in}$ du réservoir qui correspond à la zone d'entrée 1 est le même que le diamètre $D_{out}$ du réservoir qui correspond à la zone de sortie 4.

Cependant, la zone d'entrée 1 et la zone de sortie 4 peuvent avoir des diamètres et/ou des hauteurs différentes. En outre, la zone d'entrée 1 et/ou la zone de sortie 4 peuvent avoir des formes autres que cylindrique (par exemple des formes carrées).

Il est à noter que les dimensions (hauteur et diamètre) de la zone d'entrée 1 sont choisies par rapport aux dimensions de l'explant (ganglion ou hippocampe notamment) reçu par cette zone d'entrée 1, de sorte que l'échantillon biologique puisse entrer dans le dispositif microfluidique 10 de la figure 3 par cette zone d'entrée 1, comme c'est le cas pour la zone d'entrée 1 du dispositif microfluidique 10 de la figure 1.

En outre, comme c'est le cas pour la zone d'entrée 1 du dispositif microfluidique 10 de la figure 1, il est souhaitable que les dimensions de la zone d'entrée 1 du dispositif microfluidique 10 de la figure 3 soient adéquates pour pouvoir stocker la quantité des nutriments qui sont inclus dans le liquide et qui sont nécessaires à la survie de l'explant dans la zone de confinement, pour une durée pouvant être comprise entre 12 heures et 48 heures (mais non limitée à ces durées), afin d'effectuer des études *in vitro* pour ces cellules.

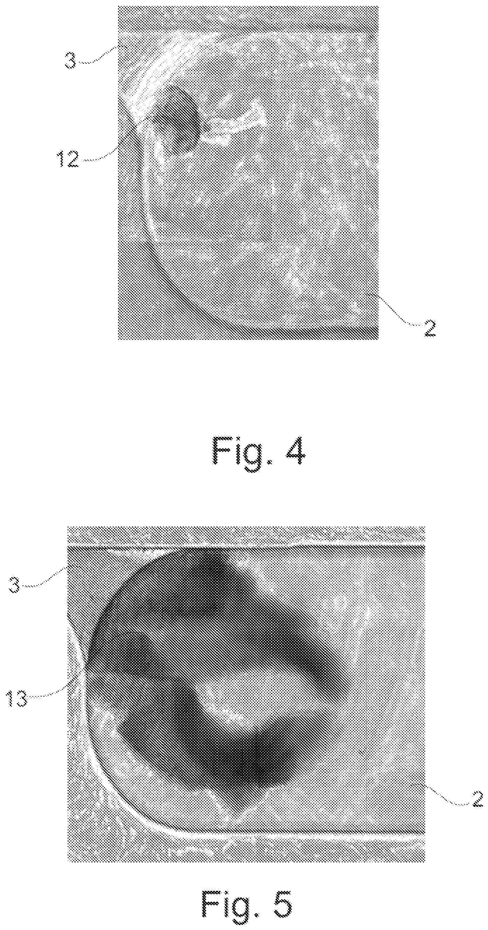

Le dispositif microfluidique 10 de la figure 3 comporte en outre une zone de confinement 5 où l'explant est confiné. Cette zone de confinement correspond à un espace du premier canal 2 (voir par exemple l'espace aval du premier canal 2 dans l'exemple des figures 4 et 5 ci-dessous) et les dimensions de cette zone de confinement 5 peuvent varier de quelques micromètres à quelques centimètres.

En outre, ce dispositif microfluidique 10 comporte un premier canal 2 et un second canal 3, qui ont des hauteurs inférieures à celles respectivement de la zone d'entrée 1 et de la zone de sortie 4. En outre, comme illustré dans la figure 3, le premier canal 2 a une hauteur $H_{in}$, une longueur $L_{in}$ et une largeur $W_{in}$ et le second canal 3 a une hauteur $H_{out}$, une longueur $L_{out}$ et une largeur $W_{out}$.

Par ailleurs, le premier canal 2 et le second canal 3 du dispositif microfluidique 10 de la figure 3 sont disposés l'un par rapport à l'autre de telle sorte que le liquide de culture cellulaire contenant l'explant introduit par la zone d'entrée 1 s'écoule via le premier canal 2, la zone de confinement puis le second canal 3 vers la zone de sortie 4.

Il est à noter que l'écoulement du liquide dans le dispositif microfluidique de la figure 3 peut être stoppé à tout moment soit en retirant le volume de liquide restant de la zone d'entrée 1, soit en rajoutant un volume de liquide équivalent dans la zone de sortie 4.

En outre, il est à noter que la zone de sortie 4 peut être adaptée pour permettre une aspiration du liquide afin d'augmenter la vitesse du liquide dans le dispositif microfluidique 10. En particulier, cette aspiration du liquide peut être effectuée en utilisant une pipette, un embout ou une capillaire d'aspiration et les dimensions de la zone de sortie 4 sont adaptées afin qu'elles correspondent aux dimensions de la pipette, de l'embout ou de la capillaire d'aspiration.

Il est à noter que comme c'est le cas pour le dispositif microfluidique 10 de la figure 1, les dimensions respectives du premier canal 2 et du second canal 3 du dispositif microfluidique 10 de la figure 3 sont adaptées pour que, lors de l'écoulement du liquide, au moins une partie de l'échantillon biologique soit confiné dans la zone de confinement 5 et que la distribution spatiale de cet échantillon biologique dans la zone de confinement 5 soit contrôlée. En particulier, la largeur $W_{out}$ et/ou la hauteur $H_{out}$ du second canal 3 est inférieure à la largeur $W_{in}$ et/ou la hauteur $H_{in}$ respective du premier canal 2 afin que le second canal 3 fonctionne comme une barrière physique pour l'échantillon biologique et ainsi, le confinement de l'échantillon biologique est effectué dans un espace du premier canal 2, comme mentionné ci-dessus. Selon un exemple, la largeur $W_{out}$ et/ou la hauteur $H_{out}$ du second canal 3, en dehors d'être inférieures à la largeur $W_{in}$ et/ou la hauteur $H_{in}$ respectives du premier canal 2, elles sont de plus inférieures aux dimensions de l'échantillon biologique afin que le second canal 3 peut offrir une barrière physique améliorée qui est adaptée aux dimensions particulières de l'échantillon biologique afin de bloquer plus efficacement son passage dans le second canal 3.

En outre, l'adaptation de la largeur et/ou la hauteur du premier canal 2 dépend des dimensions de l'explant et dans tous les cas elles sont de préférence au moins 1% plus grandes que les dimensions de l'échantillon biologique en suspension afin que l'échantillon biologique puisse traverser sans dommage le premier canal 2.

Par ailleurs, en ce qui concerne la longueur du premier canal 2, il est souhaitable qu'elle soit la plus courte possible, de manière qu'elle permette à l'explant d'être introduite dans le premier canal 2, et qu'elle empêche les accidents de parcours (accroche non voulue de l'explant aux parois du premier canal 2 avant arriver à la zone de confinement).

Dans l'exemple de la figure 3, l'élément critique pour confiner au moins une partie de l'échantillon biologique dans la zone de confinement 5 en en contrôlant da distribution spatiale dans cette zone de confinement est la structure particulière du dispositif microfluidique 10 mentionnée ci-dessus, et non la perte de charge comme c'est le cas pour l'exemple de la figure 1. Ainsi, il a été constaté que la présence du premier canal 2 et du second canal 3 dans le dispositif microfluidique 10 de la figure 3, et plus particulièrement l'adaptation adéquate décrite ci-dessus des dimensions respectives de ces deux canaux 2, 3, permet d'obtenir un dispositif microfluidique 10 dans lequel on peut contrôler la vitesse de l'écoulement du liquide dans la zone de confinement afin de confiner au moins une partie de l'échantillon biologique dans cette zone de confinement en contrôlant la distribution spatiale de cet échantillon biologique dans cette zone de confinement. Il est à noter que dans cet exemple de la figure 3, la vitesse de l'écoulement du liquide dans la zone de confinement peut être contrôlée par l'aspiration du liquide mentionnée ci-dessus.

Echantillons

L'un des échantillons mis en œuvre dans cet exemple (cas de la figure 4) est un échantillon biologique, qui comprend :

- un ganglion, en suspension dans
- un milieu liquide de culture cellulaire, contenant du Neurobasal, du b27, de la L-cystéine et du Pen/Strep.

L'autre échantillon mis en œuvre dans cet exemple (cas de la figure 5) est également un échantillon biologique, mais qui comprend :

- un un hippocampe, en suspension dans
- un milieu liquide de culture cellulaire, contenant du Neurobasal, du b27, de la L-cystéine et du Pen/Strep.

Dimensionnement de la puce microfluidique des figures 4 et 5 en fonction de la taille de l'explant

Les figures 4 et 5 illustrent chacune une photographie représentant une vue de dessus d'une zone de confinement du dispositif microfluidique 10 schématiquement illustré sur la figure 3 :

- sur la figure 4, l'expiant 12 confiné est un ganglion d'un diamètre de 250$\mu$m, et
- sur la figure 5, l'explant 13 confiné est un hippocampe d'une longueur de 3 mm et d'une largeur de 200 $\mu$m.

Comme on peut le constater sur les figures 4 et 5, la zone de confinement 5 correspond à un espace aval du premier canal 2 et la hauteur du second canal 3 est telle que le passage de l'explant dans le second canal 3 est bloqué. Il est à noter que dans l'exemple des figures 4 et 5, le canal d'entrée 2 a une hauteur de 1,2mm, une largeur de 2,3mm et une longueur de 3 mm et le canal de sortie 3 a une hauteur de 100$\mu$m, une largeur de 100 pm et une longueur de 4 mm. Par ailleurs, pour l'exemple des figures 4 et 5, la zone d'entrée (non illustrée dans les figures 4 et 5) est cylindrique et a une hauteur de 5 mm et un diamètre de 4 mm et la zone de sortie (non illustrée dans les figures 4 et 5) est aussi cylindrique et a une hauteur de 5 mm et un diamètre et 2 mm.

Dans l'exemple des figures 4 et 5, le volume de liquide introduit par la zone d'entrée 1 est 40μL et la vitesse de l'écoulement du liquide dans la zone de confinement est induite par aspiration.

## Liste des références

[1] Taylor, A. M. et al. "A microfluidic culture platform for CNS axonal injury, regeneration and transport", Nature Methods, Vol. 2, No. 8, August 2005, pages 599-605.

[2] Park, J., Koito, H., J. & Han, A. "Microfluidic compartmentalized co-culture platform for CNS axon myelination research" Biomed. Microdevices 11, 1145-1153 (2009).

[3] Shi, P., Nedelec, S., Wichterle, H. & Kam, L. C., "Combined microfluidics/protein patterning platform for pharmacological interrogation of axon pathfinding" Lab Chip 10, 1005-10 (2010).

[4] Peyrin, J. M. et al., "Axon diodes for the reconstruction of oriented neuronal network in microfluidic chambers" Lab Chip 12, 3663 (2011).

[5] Barbati, A. C., Fang, C., Banker, G. & Kirby, B. J., "Culture of primary rat hippocampal neurons: design, analysis, and optimization of a microfluidic device for cell seeding, coherent growth, and solute delivery." Biomed. Microdevices 15, 97-108 (2013).

[6] Dinh, N. D., et al., "Microfluidic construction of minimalistic neuronal co-cultures" Lab Chip 13, 1402-12 (2013).

[7] Pautot, S., Wyart, C. & Isacoff, E. Y., "Colloid-guided assembly of oriented 3D neuronal networks" Nat Meth 5, 735-740 (2008).

[8] Huang, Z. et al., "Assembly of functional Three-Dimensional Neuronal Networks on a Microchip." Small (2014) .doi:10. 1002/smll.201400513.

[9] Bang, S., Na, S., Jang, J. M., Kim, J. & Jeon, N. L. Engineering-Aligned 3D Neural Circuit in Microfluidic Device. Adv. Healthc. Mater. 5, 159-166 (2016).

[10] Kato-Negishi, M., Morimoto, Y., Onoe, H. & Takeushi, S. "millimeter-sized neural building blocks for 3D heterogeneous neural network assembly" Adv. Healthc. Mater. 2, 1564-70 (2013).

## Revendications

1. Procédé de dimensionnement d'un dispositif microfluidique (10) destiné à confiner un échantillon initial comprenant au moins une population de cellules ou de microparticules en suspension dans un milieu fluide porteur, le dispositif microfluidique (10) comportant :

   • une zone d'entrée (1) adaptée pour recevoir le milieu fluide porteur contenant l'échantillon, ladite zone d'entrée (1) correspondant à un réservoir cylindrique d'entrée de diamètre $D_{in}$,
   • une zone de confinement (5) dans laquelle au moins une partie de l'échantillon est confinée comprenant un fond (51) de surface $S_{ch}$ et de longueur $L_{ch}$ et une paroi latérale (52) de hauteur $H_{ch}$, ladite zone de confinement (5) communiquant avec ladite la zone d'entrée (1) par un premier canal (2) de longueur $L_{in}$, de hauteur $H_{in}$ et de largeur $W_{in}$, et
   • une zone de sortie (4) adaptée pour évacuer ledit liquide incluant l'échantillon, ladite zone de sortie (4) correspondant à un réservoir cylindrique de sortie de diamètre $D_{out}$, ladite zone de sortie (4) communiquant avec ladite zone de confinement (5) par un second canal (3) de longueur $L_{out}$, de hauteur $H_{out}$ et de largeur $W_{out}$,
   ledit procédé étant **caractérisé en ce qu'**il consiste en un dimensionnement dudit dispositif (10) en fonction du nombre de cellules ou de microparticules à confiner, comprenant les étapes suivantes :

A. dimensionnement de ladite zone de confinement (5) en fonction de la quantité souhaitée de cellules ou de microparticules à confiner et du taux de recouvrement φ souhaité du fond (51) de ladite zone de confinement (5) par lesdites cellules ou microparticules, de manière à définir la surface $D_{Ch}$ et la hauteur $H_{Ch}$ caractérisant la zone de confinement (5) ;

B. dimensionnement du premier canal (2) et du second canal (3) comprenant :

• b1) le calcul de la vitesse de sédimentation $v_{sedi}$ d'une particule ou d'une cellule,
• b2) détermination de la vitesse $v_{ch}$ du milieu fluide porteur dans ladite zone de confinement (5) en fonction de la vitesse de sédimentation $V_{sedi}$ d'une particule ou d'une cellule conformément à l'équation (1) :

$$V_{ch} \leq V_{sedi} \frac{H_{ch}}{D_{ch}} \ (1)$$

• b3) détermination de la perte de charge dans ledit dispositif en fonction du volume de milieu fluide injecté $\Delta Z$ entre les zones d'entrée (1) et de sortie (4) qui est nécessaire à l'établissement d'un écoulement adéquat dans la zone de confinement (5) ;
• b4) détermination des paramètres géométriques dudit dispositif microfluidique (10) à partir de $\Delta Z$ et de la vitesse $v_{ch}$ du milieu fluide porteur.

dans lequel l'étape A de dimensionnement de ladite zone de confinement (5) comprend

AI) la détermination de la surface SC h du fond de ladite zone de confinement (5) conformément à la formule de Stokes (5) :

$$\phi = \frac{N \times \pi r^2}{Sch} \ (5)$$

avec

- φ étant le taux de recouvrement φ du fond (51),
- r étant le rayon d'une particule ou d'une cellule
- N étant le nombre de neurones ou de cellules à confiner, défini par l'équation (6) :

$$N = \varphi \ X \ V \ (6)$$

avec

- φ étant la concentration en cellules ou neurones dans ledit échantillon,
- V étant le volume d'échantillon entré dans ledit dispositif microfluidique (10) à un instant t, défini conformément à l'équation (7) :

$$V = Q \ X \ t \ (7)$$

avec

- Q étant le débit de l'échantillon dans ledit dispositif microfluidique (10) .

A2) la fixation par l'utilisateur dudit dispositif microfluidique (10) de la hauteur $H_{ch}$ de la paroi de hauteur $H_{ch}$, en fonction de la quantité de volume désiré dans la zone de confinement (5) et des contraintes de fabrication associées.

2. Procédé selon la revendication 1, dans lequel la zone de confinement (5) est de géométrie cylindrique avec un fond (51) circulaire de diamètre $D_{Ch}$, de sorte que $L_{Ch} = D_{Ch}$.

3. Procédé selon l'une quelconque des revendications 1 à 2, dans lequel la vitesse de sédimentation vse di d'une particule ou d'une cellule est calculée dans la sous-étape b1) conformément à l'équation de Stokes (8) :

$$vsedi = \frac{2\,r^2 g \Delta\rho}{9\eta}\ (8)$$

avec :

- r étant le rayon d'une particule ou d'une cellule,
- $\eta$ étant la viscosité dynamique dudit milieu fluide porteur, et
- $\Delta\rho$ étant la différence de masse volumique entre celle des particules ou cellules à confiner et le milieu fluide porteur.

4. Procédé selon l'une quelconque des revendications 1 à 3, dans lequel l'étape b4) de détermination des paramètres géométriques dudit dispositif microfluidique (10) comprend les sous-étapes suivantes :

    • b41) choix de sept paramètres géométriques dudit dispositif microfluidique (10) parmi les huit paramètres géométriques $D_{in}$, $H_{in}$, $W_{in}$, $L_{in}$, $D_{out}$, $H_{out}$, $L_{out}$ et $W_{out}$; et
    • b42) calcul du paramètre géométrique inconnu restant en fonction de $\Delta Z$ et de la vitesse $v_{ch}$ du milieu fluide porteur.

5. Procédé selon la revendication 4, dans lequel le paramètre inconnu est calculé dans la sous-étape b42) à partir de l'équation (2') ci-dessous, pour une perte de charge régulière et un écoulement laminaire du milieu fluide porteur dans ledit dispositif microfluidique (10)

$$\Delta z = \frac{\eta Q}{\rho g}\left(\frac{\lambda_{in} L_{in}}{W_{in} H_{in}{}^3} + \frac{\lambda_{ch} L_{ch}}{W_{ch} H_{ch}{}^3} + \frac{\lambda_{out} L_{out}}{W_{out} H_{out}{}^3}\right)\ (10)$$

avec :

- Q étant le débit de fluide constant dans ledit dispositif (10) défini par l'équation (9) :

$$Q = v_{ch} \times H_{ch} \times W_{ch}\ (9)$$

- g désigne l'accélération gravitationnelle,
- $\eta$ désigne la viscosité dynamique du milieu fluide porteur,
- $\rho$ désigne la masse volumique du milieu fluide porteur,
- $L_{ch}$ désigne la largeur de la zone de confinement (5),
- $\lambda$ représente un coefficient de frottement, calculé pour un faible nombre de Reynolds par l'équation (3) :

$$\lambda = 12\left(1 - 6\left(\frac{2}{\pi}\right)^5\left(\frac{H}{W}\right)\right)(3)$$

6. Procédé selon la revendication 5, dans lequel le paramètre inconnu est $L_{out}$ qui est calculé selon l'équation (4) :

$$L_{out} = \frac{W_{out} H_{out}{}^3}{\lambda_{out}}\left(\Delta Z \frac{\rho g}{\eta Q} - \frac{\lambda_{in} L_{in}}{W_{in} H_{in}{}^3} - \frac{\lambda_{ch} L_{ch}}{W_{ch} H_{ch}{}^3}\right)(4)$$

7. Procédé selon l'une quelconque des revendications 1 à 6, dans lequel l'échantillon est un échantillon biologique consistant en une population de cellules choisies parmi les neurones et les cellules eucaryotes en suspension dans un milieu de culture cellulaire, ou en suspension dans de l'eau, salée ou non, un solvant, un hydrogel ou un échafaudage organique ou un polymère.

8. Procédé selon l'une quelconque des revendications 1 à 6, dans lequel l'échantillon est un échantillon non- biologique consistant en une population de microparticules en suspension dans de l'eau, salée ou non, un solvant, un hydrogel ou un échafaudage organique ou un polymère, lesdites microparticules étant choisies parmi les microparticules métalliques, ou en matériau semi-conducteur, ou en polyéthylène-glycol (PEG).

**9.** Procédé selon l'une quelconque des revendications 1 à 8, dans lequel on relie ladite zone de confinement (5) à au moins une chambre d'isolation (7), par au moins un canal additionnel (6) ayant une résistance hydraulique permettant le passage, sans retour, de l'échantillon dans ladite chambre d'isolâtion (7) .

**10.** Procédé selon la revendication 9, dans lequel ladite chambre d'isolation (7) est une zone de confinement d'un dispositif microfluidique additionnel.

**11.** Procédé selon les revendications 9 ou 10, dans lequel ladite chambre d'isolation comporte un échantillon additionnel.

**12.** Utilisation d'un dispositif microfluidique (10) tel que dimensionné selon la revendication 11, pour étudier l'interaction entre l'échantillon initial dans ladite zone de confinement (5) et l'échantillon additionnel dans ladite chambre d'isolation (6).

**Patentansprüche**

**1.** Verfahren zur Dimensionierung einer mikrofluidischen Vorrichtung (10), die dazu vorgesehen ist, eine anfängliche Probe, umfassend mindestens eine Population von Zellen oder Mikroteilchen, in Suspension in einem Trägerflüssigkeitsmedium einzugrenzen, wobei die mikrofluidische Vorrichtung (10) umfasst:

• eine Eintrittszone (1), die dazu eingerichtet ist, das Trägerflüssigkeitsmedium, enthaltend die Probe, aufzunehmen, wobei die Eintrittszone (1) einem zylindrischen Eintrittsreservoir mit einem Durchmesser $D_{in}$ entspricht,
• eine Eingrenzungszone (5), wobei mindestens ein Teil der Probe eingegrenzt ist, umfassend einen Boden (51) mit einer Oberfläche $S_{ch}$ und einer Länge $L_{ch}$ und einer Seitenwand (52) mit einer Höhe $H_{Ch}$, wobei die Eingrenzungszone (5) durch einen ersten Kanal (2) mit einer Länge $L_{in}$, einer Höhe $H_{in}$ und einer Breite $W_{in}$ mit der Eintrittszone (1) in Verbindung steht, und
• eine Austrittszone (4), die dazu eingerichtet ist, die Flüssigkeit, beinhaltend die Probe, abzuführen, wobei die Austrittszone (4) einem zylindrischen Austrittsreservoir mit einem Durchmesser $D_{out}$ entspricht, wobei die Austrittszone (4) durch einen zweiten Kanal (3) mit einer Länge $L_{out}$, einer Höhe $H_{out}$ und einer Breite $W_{out}$ mit der Eingrenzungszone (5) in Verbindung steht, wobei das Verfahren **dadurch gekennzeichnet ist, dass** es aus einer Dimensionierung der Vorrichtung (10) in Abhängigkeit von der Anzahl von einzugrenzenden Zellen oder Mikroteilchen besteht, umfassend die folgenden Schritte:

A. Dimensionierung der Eingrenzungszone (5) in Abhängigkeit von der gewünschten Quantität von einzugrenzenden Zellen oder Mikroteilchen und dem gewünschten Abdeckungsgrad φ des Bodens (51) der Eingrenzungszone (5) durch die Zellen oder Mikroteilchen, um die Oberfläche $D_{Ch}$ und die Höhe $H_{Ch}$, die die Eingrenzungszone (5) charakterisieren, zu definieren;
B. Dimensionierung des ersten Kanals (2) und des zweiten Kanals (3), umfassend:

• b1) Berechnung der Absetzgeschwindigkeit $V_{sedi}$ eines Teilchens oder einer Zelle,
• b2) Bestimmung der Geschwindigkeit $v_{ch}$ des Trägerflüssigkeitsmediums in der Eingrenzungszone (5) in Abhängigkeit von der Absetzgeschwindigkeit $V_{sedi}$ eines Teilchens oder einer Zelle gemäß der Gleichung (1):

$$v_{ch} \le v_{sedi} \frac{H_{ch}}{D_{ch}} \quad (1) \, ,$$

• b3) Bestimmung des Druckverlusts in der Vorrichtung in Abhängigkeit von dem Volumen des injizierten Flüssigkeitsmediums $\Delta Z$ zwischen der Eintrittszone (1) und der Austrittszone (4), das zur Einrichtung eines adäquaten Flusses in der Eingrenzungszone (5) erforderlich ist;
• b4) Bestimmung von geometrischen Parametern der mikrofluidischen Vorrichtung (10) aus $\Delta Z$ und der Geschwindigkeit $v_{ch}$ des Trägerflüssigkeitsmediums,
wobei der Schritt A zur Dimensionierung der Eingrenzungszone (5) umfasst:
A1) Bestimmung der Oberfläche SCh des Bodens der Eingrenzungszone (5) gemäß der Stokes-Formel (5):

$$\phi = \frac{N x \pi r^2}{S_{ch}} \quad (5) \, ,$$

wobei

- $\varphi$ der Abdeckungsgrad $\varphi$ des Bodens (51) ist,
- r der Radius eines Teilchens oder einer Zelle ist,
- N die Anzahl von einzugrenzenden Neuronen oder Zellen ist, definiert durch die Gleichung (6):

$$N \ = \ \varphi \ X \ V \ (6),$$

wobei
- $\varphi$ die Konzentration von Zellen oder Neuronen in der Probe ist,
- V das Volumen der Probe, das in die mikrofluidische Vorrichtung (10) eintritt, zu einem Zeitpunkt t, definiert gemäß der Gleichung (7):

$$V \ = \ Q \ X \ t \ (7),$$

wobei
- Q der Durchsatz der Probe in der mikrofluidischen Vorrichtung (10) ist,
A2) Festlegung der Höhe $H_{ch}$ der Wand mit der Höhe $H_{ch}$ in Abhängigkeit von der gewünschten Volumenquantität in der Eingrenzungszone (5) und assoziierten Herstellungseinschränkungen durch den Benutzer der mikrofluidischen Vorrichtung (10).

2.  Verfahren nach Anspruch 1, wobei die Eingrenzungszone (5) eine zylindrische Geometrie mit einem kreisförmigen Boden (51) mit einem Durchmesser $D_{Ch}$, aufweist, so dass $L_{Ch} = D_{Ch}$.

3.  Verfahren nach einem der Ansprüche 1 bis 2, wobei die Absetzgeschwindigkeit vse eines Teilchens oder einer Zelle im Unterschritt b1) gemäß der Stokes-Gleichung (8) berechnet wird:

$$vsedi = \frac{2r^2 g \Delta \rho}{9\eta} \quad (8),$$

wobei:

- r der Radius eines Teilchens oder einer Zelle ist,
- $\eta$ die dynamische Viskosität des Trägerflüssigkeitsmediums ist und
- $\Delta\rho$ der Unterschied der Dichte zwischen der von einzugrenzenden Teilchen oder Zellen und des Trägerflüssigkeitsmediums ist.

4.  Verfahren nach einem der Ansprüche 1 bis 3, wobei der Schritt b4) zur Bestimmung von geometrischen Parametern der mikrofluidischen Vorrichtung (10) die folgenden Unterschritte umfasst:

    • b41) Auswahl von sieben geometrischen Parametern der mikrofluidischen Vorrichtung (10) aus den acht geometrischen Parametern $D_{in}$, $H_{in}$, $W_{in}$, $L_{in}$, $D_{out}$, $H_{out}$, $L_{out}$ und $W_{out}$ und
    • b42) Berechnung des verbleibenden unbekannten geometrischen Parameters in Abhängigkeit von $\Delta Z$ und der Geschwindigkeit $v_{ch}$ des Trägerflüssigkeitsmediums.

5.  Verfahren nach Anspruch 4, wobei der unbekannte Parameter im Unterschritt b42) aus der folgenden Gleichung (2') für einen regulären Druckverlust und einen Laminarfluss des Trägerflüssigkeitsmediums in der mikrofluidischen Vorrichtung (10) berechnet wird:

$$\Delta Z = \frac{\eta Q}{\rho g} \left( \frac{\lambda_{in} L_{in}}{W_{in} H_{in}^{3}} + \frac{\lambda_{ch} L_{ch}}{W_{ch} H_{ch}^{3}} + \frac{\lambda_{out} L_{out}}{W_{out} H_{out}^{3}} \right) \quad (10),$$

wobei:

- Q der konstante Flüssigkeitsdurchsatz in der Vorrichtung (10) ist, definiert durch die Gleichung (9):

$$Q = v_{ch} \times H_{ch} \times W_{ch} \quad (9),$$

- g das Schwerefeld bezeichnet,
- η die dynamische Viskosität des Trägerflüssigkeitsmediums bezeichnet,
- ρ die Dichte des Trägerflüssigkeitsmediums bezeichnet,
- $L_{ch}$ die Breite der Eingrenzungszone (5) bezeichnet,
- λ für einen Reibungskoeffizienten steht, berechnet für eine niedrige Reynolds-Zahl durch die Gleichung (3):

$$\lambda = 12\left(1 - 6\left(\frac{2}{\pi}\right)^5\left(\frac{H}{W}\right)\right) \quad (3).$$

**6.** Verfahren nach Anspruch 5, wobei der unbekannte Parameter $L_{out}$ ist, der gemäß der Gleichung (4) berechnet wird:

$$L_{out} = \frac{W_{out} H_{out}^{3}}{\lambda_{out}}\left( \Delta z \frac{\rho g}{\eta Q} - \frac{\lambda_{in} L_{in}}{W_{in} H_{in}^{3}} - \frac{\lambda_{ch} L_{ch}}{W_{ch} H_{ch}^{3}} \right) \quad (4).$$

**7.** Verfahren nach einem der Ansprüche 1 bis 6, wobei die Probe eine biologische Probe ist, bestehend aus einer Population von Zellen, die aus Neuronen und eukaryotischen Zellen ausgewählt sind, in Suspension in einem Zellkulturmedium oder in Suspension in Wasser, salzhaltig oder nicht, einem Lösungsmittel, einem Hydrogel oder einem organischen Gerüst oder einem Polymer.

**8.** Verfahren nach einem der Ansprüche 1 bis 6, wobei die Probe eine nichtbiologische Probe ist, bestehend aus einer Population von Mikroteilchen in Suspension in Wasser, salzhaltig oder nicht, einem Lösungsmittel, einem Hydrogel oder einem organischen Gerüst oder einem Polymer, wobei die Mikroteilchen aus metallischen Mikroteilchen oder aus einem Halbleitermaterial oder aus Polyethylenglykol (PEG) ausgewählt sind.

**9.** Verfahren nach einem der Ansprüche 1 bis 8, wobei die Eingrenzungszone (5) mit mindestens einer Isolationskammer (7) durch mindestens einen zusätzlichen Kanal (6) mit einem hydraulischen Widerstand, der das Hindurchtreten der Probe in der Isolationskammer (7) ohne Rücklauf zulässt, verbunden wird.

**10.** Verfahren nach Anspruch 9, wobei die Isolationskammer (7) eine Eingrenzungszone einer zusätzlichen mikrofluidischen Vorrichtung ist.

**11.** Verfahren nach den Ansprüchen 9 oder 10, wobei die Isolationskammer eine zusätzliche Probe umfasst.

**12.** Verwendung einer wie gemäß Anspruch 11 dimensionierten mikrofluidischen Vorrichtung (10) zum Untersuchen der Wechselwirkung zwischen der anfänglichen Probe in der Eingrenzungszone (5) und der zusätzlichen Probe in der Isolationskammer (6).

## Claims

**1.** Method for sizing a microfluidic device (10) intended to confine an initial sample comprising at least one population of cells or microparticles in suspension in a carrier fluid medium, the microfluidic device (10) including:

• an input zone (1) adapted to receive the carrier fluid medium containing the sample, said input zone (1) corresponding to a cylindrical input tank of diameter $D_{in}$,
• a confinement zone (5) wherein at least one part of the sample is confined comprising a base (51) of surface area $S_{ch}$ and length $L_{ch}$ and a side wall (52) of height $H_{ch}$, said confinement zone (5) communicating with said input zone (1) via a first channel (2) of length $L_{in}$, height $H_{in}$ and width $W_{in}$, and
• an output zone (4) adapted to discharge said liquid including the sample, said output zone (4) corresponding to a cylindrical output tank of diameter $D_{out}$, said output zone (4) communicating with said confinement zone (5) via a second channel (3) of length $L_{out}$, height $H_{out}$ and width $W_{out}$,
said method being **characterised in that** it consists of a sizing of said device (10) according to the number of cells or microparticles to be confined, comprising the following steps:

A. sizing said confinement zone (5) according to the sought quantity of cells or microparticles to be confined and the sought coverage rate $\varphi$ of the base (51) of said confinement zone (5) by said cells or microparticles, so as to define the surface area $D_{Ch}$ and the height $H_{Ch}$ characterising the confinement zone (5);
B. sizing the first channel (2) and the second channel (3) comprising:

• b1) calculating the sedimentation rate $V_{sedi}$ of a particle or a cell,
• b2) determining the velocity $v_{ch}$ of the carrier fluid medium in said confinement zone (5) according to the sedimentation rate $V_{sedi}$ of a particle or a cell according to the equation (1):

$$V_{ch} \leq V_{sedi} \frac{H_{ch}}{D_{ch}} \ (1)$$

• b3) determining the head loss in said device according to the injected fluid medium volume $\Delta Z$ between the input (1) and output (4) zones which is necessary to establish an adequate flow in the confinement zone (5);

b4) determining the geometric parameters of said microfluidic device (10) based on $\Delta Z$ and the velocity $v_{ch}$ of the carrier fluid medium,
wherein step A of sizing said confinement zone (5) comprises:
A1) determining the surface area SCh of the base of said confinement zone (5) according to Stokes' formula (5):

$$\phi = \frac{N \times \pi r^2}{Sch} \ (5)$$

where

- $\varphi$ is the coverage rate $\varphi$ of the base (51),
- r is the radius of a particle or a cell
- N is the number of neurons or cells to be confined, defined by equation (6):

$$N = \varphi \ X \ V \ (6)$$

where
- $\varphi$ is the concentration of cells or neurons in said sample,
- V is the sample volume entered in said microfluidic device (10) at a time t, defined according to equation (7):

$$V = Q \ X \ t \ (7)$$

where
- Q is the flow rate of the sample in said microfluidic device (10),
A2) setting by the user of said microfluidic device (10) of the height $H_{ch}$ of the wall of height $H_{Ch}$, according to the sought quantity of volume in the confinement zone (5) and associated manufacturing constraints.

2. Method according to claim 1, wherein the confinement zone (5) is of cylindrical geometry with a circular base (51) of diameter $D_{Ch}$, such that $L_{Ch} = D_{Ch}$.

3. Method according to any one of claims 1 to 2, wherein the sedimentation rate vsedi of a particle or a cell is calculated in sub-step b1) according to Stokes' equation (8):

$$vsedi = \frac{2\,r^2 g \Delta\rho}{9\eta}\ (8)$$

where:

- r is the radius of a particle or a cell,
- $\eta$ is the dynamic viscosity of said carrier fluid medium, and
- $\Delta\rho$ is the difference in density between that of the particles or cells to be confined and the carrier fluid medium.

4. Method according to any one of claims 1 to 3, wherein step b4) of determining the geometric parameters of said microfluidic device (10) comprises the following sub-steps:

• b41) choosing seven geometric parameters of said microfluidic device (10) among the eight geometric parameters $D_{in}$, $H_{in}$, $W_{in}$, $L_{in}$, $D_{out}$, $H_{out}$, $L_{out}$ and $W_{out}$; and
• b42) calculating the remaining unknown geometric parameter according to $\Delta Z$ and the velocity $v_{ch}$ of the carrier fluid medium.

5. Method according to claim 4, wherein the unknown parameter is calculated in sub-step b42) from equation (2') hereinbelow, for a regular head loss and a laminar flow of the carrier fluid medium in said microfluidic device (10):

$$\Delta z = \frac{\eta Q}{\rho g}\left(\frac{\lambda_{in}L_{in}}{W_{in}H_{in}{}^3} + \frac{\lambda_{ch}L_{ch}}{W_{ch}H_{ch}{}^3} + \frac{\lambda_{out}L_{out}}{W_{out}H_{out}{}^3}\right)\ (10)$$

where:

- Q is the constant fluid flow rate in said device (10) defined by equation (9):

$$Q = V_{ch}\ x\ H_{ch}\ x\ W_{ch}\ (9)$$

- g denotes the gravitational acceleration,
- $\eta$ denotes the dynamic viscosity of the carrier fluid medium,
- $\rho$ denotes the density of the carrier fluid medium,
- $L_{ch}$ denotes the width of the confinement zone (5),
- $\lambda$ is a friction coefficient, calculated for a low Reynolds number with equation (3):

$$\lambda = 12(1 - 6(\frac{2}{\pi})^5(\frac{H}{W}))(3)$$

6. Method according to claim 5, wherein the unknown parameter is $L_{out}$ which is calculated according to equation (4):

$$L_{out} = \frac{W_{out}H_{out}{}^3}{\lambda_{out}}\left(\Delta z\frac{\rho g}{\eta Q} - \frac{\lambda_{in}L_{in}}{W_{in}H_{in}{}^3} - \frac{\lambda_{ch}L_{ch}}{W_{ch}H_{ch}{}^3}\right)(4)$$

7. Method according to any one of claims 1 to 6, wherein the sample is a biological sample consisting of a population of cells chosen from neurons and eukaryotic cells in suspension in a cellular culture medium, or in suspension in water, optionally salted, a solvent, a hydrogel or an organic scaffold or a polymer.

8. Method according to any one of claims 1 to 6, wherein the sample is a non-biological sample consisting of a population of microparticles in suspension in water, optionally salted, a solvent, a hydrogel or an organic scaffold or a polymer, said microparticles being chosen from metallic microparticles, or made of semi-conductive material, or polyethylene glycol (PEG).

9. Method according to any one of claims 1 to 8, wherein said confinement zone (5) is connected to at least one isolation chamber (7), via at least one additional channel (6) having a hydraulic resistance enabling the passage, with no return, of the sample in said isolation chamber (7).

10. Method according to claim 9, wherein said isolation chamber (7) is a confinement zone of an additional microfluidic device.

11. Method according to claims 9 or 10, wherein said isolation chamber includes an additional sample.

12. Use of a microfluidic device (10) as sized according to claim 11, for studying the interaction between the initial sample in said confinement zone(5) and the additional sample in said isolation chamber (6).

Fig. 1

Fig. 2

Fig. 3

Fig. 4

Fig. 5

## RÉFÉRENCES CITÉES DANS LA DESCRIPTION

**Documents brevets cités dans la description**

- JP 2012120474 A

**Littérature non-brevet citée dans la description**

- **TAYLOR, A. M. et al.** A microfluidic culture platform for CNS axonal injury, regeneration and transport. *Nature Methods,* Août 2005, vol. 2 (8), 599-605
- **PARK, J. ; KOITO, H., J. ; HAN, A.** Microfluidic compartmentalized co-culture platform for CNS axon myelination research. *Biomed. Microdevices,* 2009, vol. 11, 1145-1153
- **SHI, P. ; NEDELEC, S. ; WICHTERLE, H. ; KAM, L. C.** Combined microfluidics/protein patterning platform for pharmacological interrogation of axon pathfinding. *Lab Chip,* 2010, vol. 10, 1005-10
- **PEYRIN, J. M. et al.** Axon diodes for the reconstruction of oriented neuronal network in microfluidic chambers. *Lab Chip,* 2011, vol. 12, 3663
- **BARBATI, A. C. ; FANG, C. ; BANKER, G. ; KIRBY, B. J.** Culture of primary rat hippocampal neurons: design, analysis, and optimization of a microfluidic device for cell seeding, coherent growth, and solute delivery. *Biomed. Microdevices,* 2013, vol. 15, 97-108
- **DINH, N. D. et al.** Microfluidic construction of minimalistic neuronal co-cultures. *Lab Chip,* 2013, vol. 13, 1402-12
- **PAUTOT, S. ; WYART, C. ; ISACOFF, E. Y.** Colloid-guided assembly of oriented 3D neuronal networks. *Nat Meth,* 2008, vol. 5, 735-740
- **HUANG, Z. et al.** Assembly of functional Three-Dimensional Neuronal Networks on a Microchip. *Small,* 2014
- **BANG, S. ; NA, S. ; JANG, J. M. ; KIM, J. ; JEON, N. L.** Engineering-Aligned 3D Neural Circuit in Microfluidic Device. *Adv. Healthc. Mater.,* 2016, vol. 5, 159-166
- **KATO-NEGISHI, M. ; MORIMOTO, Y. ; ONOE, H. ; TAKEUSHI, S.** millimeter-sized neural building blocks for 3D heterogeneous neural network assembly. *Adv. Healthc. Mater.,* 2013, vol. 2, 1564-70